# EUROPEAN PATENT APPLICATION

(11) **EP 1 978 020 A1**
(43) Date of publication of application: **08.10.2008**
(21) Application number: 07105519.8
(22) Date of filing: 03.04.2007
(51) Int. Cl.: C07D 239/42, C07D 309/30, C07H 3/02, A61K 31/506

(54) **Processes for the preparation of statins, particularly rosuvastatin, and intermediates for the preparation thereof**

(71) Applicant: LEK Pharmaceuticals d.d., 1526 Ljubljana (SI)
(72) Inventor: Casar, Zdenko, 1370 Logatec (SI); Mesar, Tomaz, 1236 Trzin (SI)
(74) Representative: Kröger, Bernd

(57) **Abstract**

The process for synthesis of statins featuring the use of an early intermediate (4*R*,6*S*)-6-(dialkoxymethyl)tetrahydro-2*H*-pyran-2,4-diol which already possesses the desired stereochemistry corresponding to final statin.

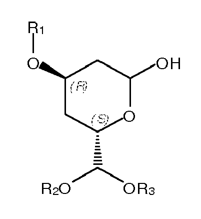

## Description

### Field of the Invention

The present invention relates in general to the field of chemical technology and in particular to a process for the preparation of HMG-CoA reductase inhibitors, known also as statins, particularly to rosuvastatin. Specifically this invention relates to preparation of a common intermediate which can be used for preparation of all statins.

### Background of the Invention

Statins, of which the representative examples may be selected from rosuvastatin, cerivastatin, atorvastatin, fluvastatin, pitavastatin, bervastatin, dalvastatin or their analogs or pravastatin, simvastatin, lovastatin or their analogs share a characteristic structure, consisting of respectively a heptenoic or heptanoic acid moiety (free acid, salt or lactone) connected to the aromatic or alicyclic core. Biological activity of statins is closely related to their stereochemistry, especially configuration at the chiral atoms of said heptenoic or heptanoic acid moiety.

### Disclosure of the Invention

In general, the present invention is directed to a process for preparing statins, in particular to the synthesis of rosuvastatin, wherein one aspect of the invention is an early intermediate, possesses the desired stereochemistry corresponding to final statin, which is a compound of formula 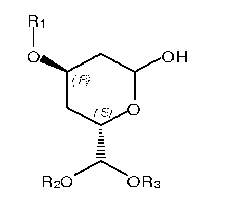 where R₁ is H or protecting group; and R₂ and R₃ are independently selected from C1 - C4 alkyl, or together form a cyclic structure of formula (CH₂)ₙ where n is from 2 to 6; in particular where R₁ is H or *tert*-butyldimethylsilyl, and R₂ and R₃ are methyl.

The overall synthetic aspect of the invention is a process for manufacturing of rosuvastatin
characterized in that it comprises following steps:
a) providing compound of formula: 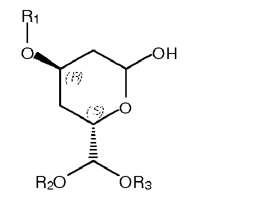 where R₁ is H or protecting group; and
   R₂ and R₃ are independently selected from C1 - C4 alkyl, or together form a cyclic structure of formula (CH₂)ₙ where n is from 2 to 6;
b) converting said compound into compound of formula 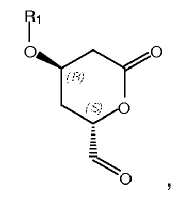 where R₁ is a protecting group; and
c) reacting said compound obtained in step b) under condition of Wittig coupling with compound of formula 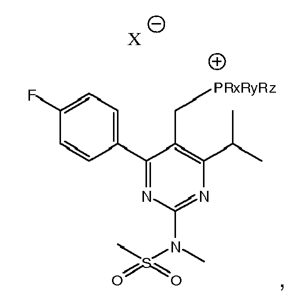 wherein Rx, Ry, and Rz, are the same or different and are selected from optionally substituted C₁ - C₈ alkyl or C₃-C₆ cycloalkyl or C₁ - C₈ alkenyl or C₅-C₆ cycloalkenyl or aryl,
   and X is an anion, preferably halogen or RCOO⁻ anion, more preferably chloride, bromide or trifluoroacetate;
to give compound of formula: 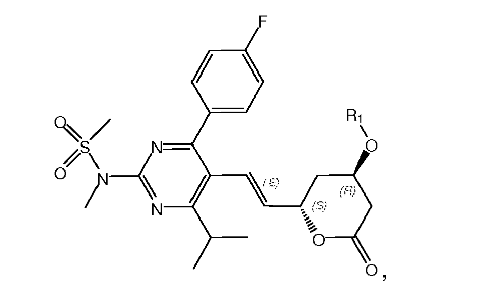 and subsequently converting said compound to rosuvastatin or its salt.

General overall synthetic aspect of the invention is a process for manufacturing of statin characterized in that it comprises following steps:
a) providing compound of formula: 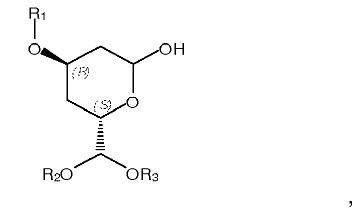 where R₁ is H or protecting group; and R₂ and R₃ are independently selected from C1 - C4 alkyl, or together form a cyclic structure of formula (CH₂)ₙ where n is from 2 to 6;
b) converting said compound into compound of formula 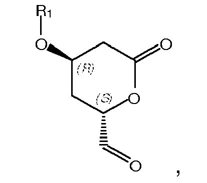 where R₁ is a protecting group; and
c) reacting said compound obtained in step b) under condition of Wittig coupling with phosphonium salt, phosphinoxide or phosphonate of the heterocyclic or alicyclic skeleton of statin,
and subsequently converting said compound obtained in step c) to statin or its salt.

Another aspect of the invention is a process for preparing compound of formula 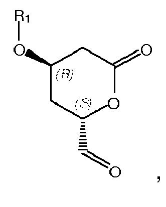 where R₁ is a protecting group
comprising the following steps:
a) converting compound of formula 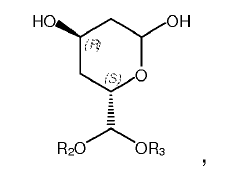 where R₂ and R₃ are independently selected from C1-C4 alkyl or together form a cyclic structure of formula (CH₂)ₙ where n is from 2 to 6,
   into compound of formula: 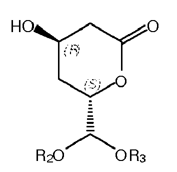
b) subsequently converting said compound obtained in step a) into compound of formula: 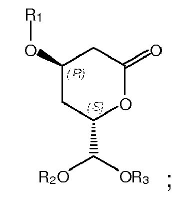 where R₁ is a protecting group, and
c) subsequently cleaving acetal in compound obtained in step b);
   wherein in particular aspect R₂ and R₃ are methyl and R₁ is silyl protecting group.

Another aspect of the invention is a process or preparing the compound of formula 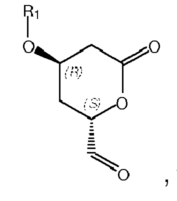 where R₁ is a protecting group,
characterized by providing the compound of formula 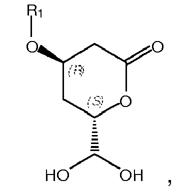 where R₁ is a protecting group,
dissolved in a solvent selected from the group of aromatic hydrocarbons, aliphatic hydrocarbons, chlorinated aromatic or aliphatic hydrocarbons and aliphatic ethers; wherein in particular aspect the solvent is selected from toluene, heptane, hexane, cyclohexane, methylcyclohexane, pentane, dichloromethane, chloroform, 2-chlorotoluene, 3-chlorotoluene, 4-chlorotoluene ^{t}BuMeO, Et₂O, Me₂O and THF. After the starting compound is provided dissolved in afformentioned solvent, the dehydration takes place and prepared compound can be isolated or used without isolation.

In particular aspect the compound 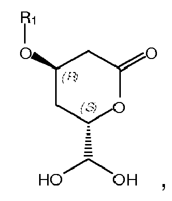 obtained from 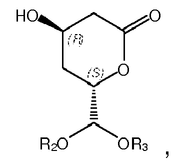 wherein R₁ is H or protecting group; and R₂ and R₃ are independently selected from C1 - C4 alkyl, or together form a cyclic structure of formula (CH₂)ₙ where n is from 2 to 6; in particular where R₁ is H or *tert-*butyldimethylsilyl, and R₂ and R₃ are methyl;
is not isolated before reaction under condition of Wittig coupling with a heterocylic or alicyclic skeleton of a statin.

Yet another aspect of the invention is a process for preparing a HMG CoA reductase inhibitor (in particular rosuvastatin), characterized in that the compound of formula 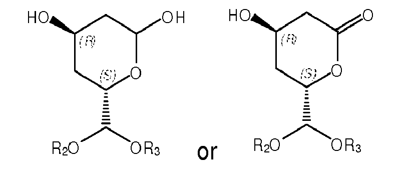 where R₂ and R₃ are independently selected from C1 - C4 alkyl or together form a cyclic structure of formula (CH₂)ₙ where n is from 2 to 6 (in particular where R₂ and R₃ are methyl), is used.

The invention additional provides for the use of any of the compounds of formula 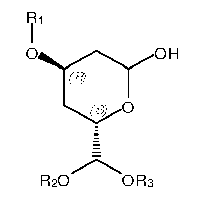 where R₁ is H or protecting group; and
R₂ and R₃ are independently selected from C1 - C4 alkyl or together form a cyclic structure of formula (CH₂)ₙ where n is from 2 to 6 (in particular where R₂ and R₃ are methyl)
for the synthesis of compound of formula 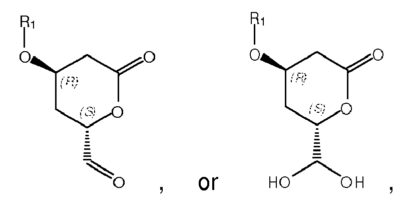 where R₁ is H or protecting group.

The aspect of the invention is use of toluene as the solvent in the Wittig reaction of the compounds of formula 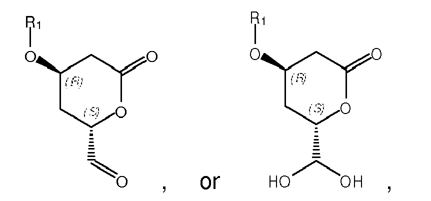 where R₁ is H or protecting group
with appropriate phosphonium salt, phosphinoxide or phosphonate.

### Detailed description of the Invention

The present invention relates in general to the synthesis of statin where the moiety FL' 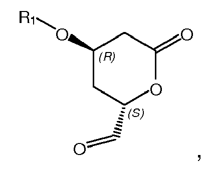 where R₁ is a protecting group; is reacted with an appropriate phosphonium salt, phosphinoxide or phosphonate of the heterocyclic or alicyclic skeleton of statin, such as with: 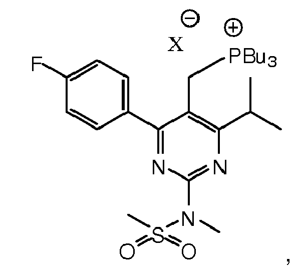 where X is suitable anion.

The compound **FL'** (in particular **FL** where R₁ is *tert*-butyldimetylsilyl) is prepared from intermediate **AVL'** (R₂ and R₃ are independently selected from C1 - C4 alkyl, or together form a cyclic structure of formula (CH₂)ₙ where n is from 2 to 6) in accordance with following general Scheme 1, or specific on Scheme 2 for **AVL** wherein R₂ and R₃ are both Me:

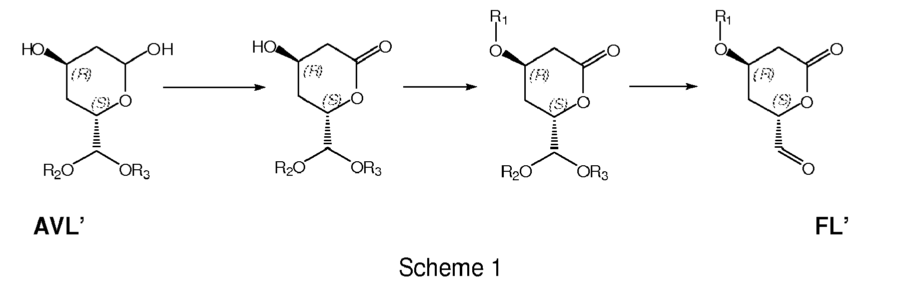

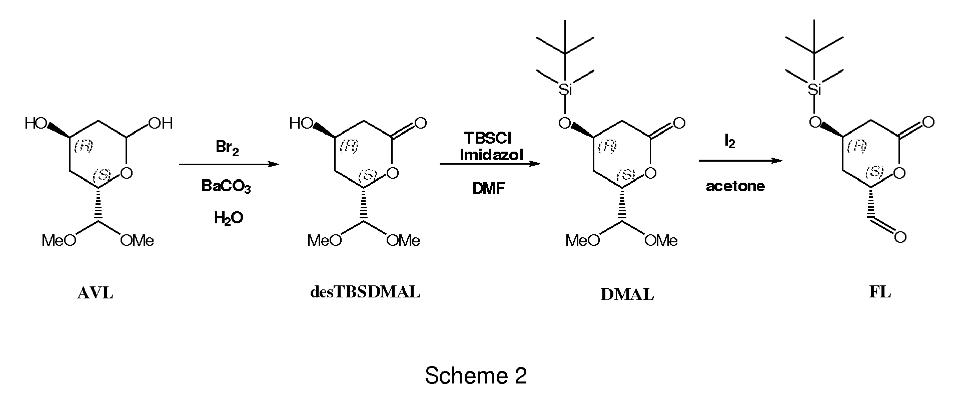

The protecting group R₁ of our invention may be any conventionally used protecting group, in particular alkyl, acyl, silyl or similar group, more particularly selected from acetyl (Ac), pivaloyl (Piv), *p*-toluenesulfonyl (TOS), β-methoxyethoxymethyl ether (MEM), methoxymethyl ether (MOM), *p*-methoxybenzyl ether (PMB), methylthiomethyl ether, *t-*butyl, tetrahydropyranyl (THP), benzyl (Bn), diphenylmethyl or triphenylmethyl group, preferably silyl protecting group, which can be represented by formula SiR₁R₂R₃ in which R₁, R₂, R₃ are independently selected from alkyl (preferably C₁-C₆) or aryl (preferably C₅-C₁₀), such as SiMe₃ (TMS), SiMe₂*t*-Bu (TBDMS), Si(*i*-Pr)₃ (TIPS), SiPh₂*t*-Bu, SiMe₂Ph.

The feature of an early intermediate **AVL',** which is chemically (4R,6S)-6-(dialkoxymethyl)tetrahydro-2*H*-pyran-2,4-diol or (4*R*,6*S*)-6-(alkylenedioxy)tetrahydro-2*H-*pyran-2,4-diol and in particular **AVL,** which is chemically (4*R*,6*S*)-6-(dimethoxymethyl)tetrahydro-2*H*-pyran-2,4-diol is that is possesses the desired stereochemistry, avoiding subsequent separations of later intermediates. On the other hand **AVL'** or **AVL** is chemically aldehyde acetal which behaves as a masked (protected) aldehyde what eliminates the necessity for the oxidation synthetic step in the synthesis of statins.

Compounds **FL'** and **FL**, or hydrate thereof, obtained from **AVL** or **AVL'** can be further used to prepare rosuvastatin as outlined on Scheme 3:

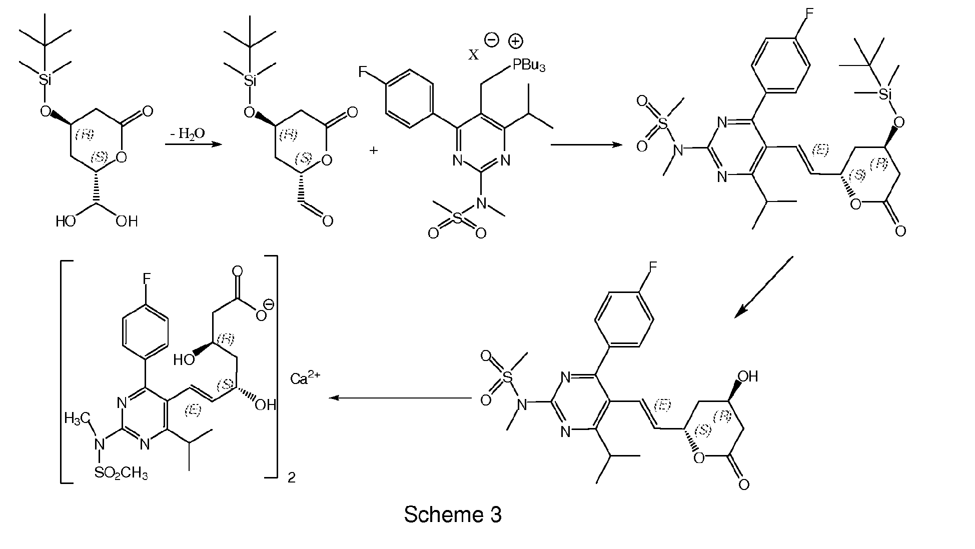

If compounds **FL'** or **FL**, or hydrates thereof in suitable solvent, are reacted under condition of Wittig coupling with a heterocylic or alicyclic derivatives (skeleton of statin) of following formula : 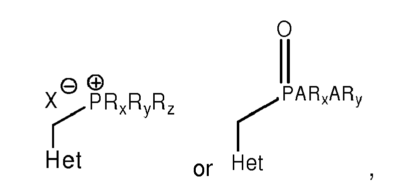 where A can be a bond or O;
and wherein Rₓ, R_{y}, and R_{z}, are the same or different and are selected from optionally substituted C₁ - C₈ alkyl or C₃-C₆ cycloalkyl or C₁ - C₈ alkenyl or C₅-C₆ cycloalkenyl or aryl;
and X is an anion, preferably halogen or RCOO- anion, more preferably chloride, bromide or trifluoroacetate;
and Het is selected so that it forms a heterocyclic or alicyclic skeleton of a statin; other HMG-CoA reductase inhibitors (preferably selected among cerivastatin, fluvastatin, pitavastatin, bervastatin, dalvastatin) can be analogously prepared.

Heterocyclic or alicyclic skeleton of statin is in particular selected from:

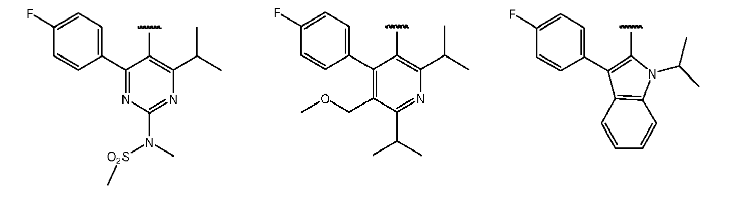

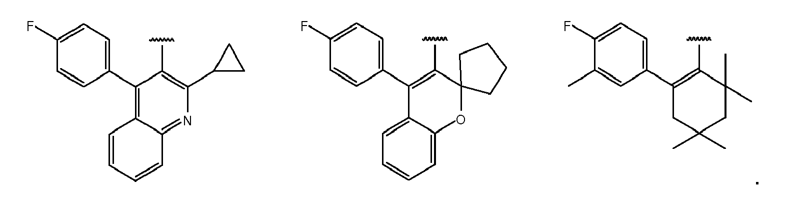

Compound **FL'** or **FL** may exist in presence of water, if in liquid state or dissolved in organic solvent in an equilibrium with its hydrated form 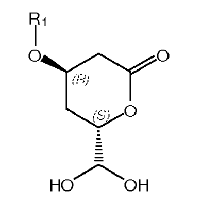 and may be isolated in aldehyde or hydrate form. Aldehyde form can be isolated from anhydrous media by evaporation while hydrate form can be isolated from solvents containing water by evaporation or precipitation and filtration. The hydrate form is preferable for isolation and storage. Witting reaction proceeds with the aldehyde form, however both forms can be used if the reaction is carried out in a solvent in which the aldehyde form is favoured in the equilibrium. In chlorinated hydrocarbons (such as chloroform, dichloromethane), hydrocarbons (such as hexane and cyclohexane) and particularly in aromatic hydrocarbons (such as toluene or its chlorinated analogues) the equilibrium is shifted completely towards aldehyde. Use of toluene as the solvent for Wittig reaction (with phosphonium salt, phosphinoxide or phosphonate of heterocyclic or alicyclic skeleton of a statin) significantly increases the yield compared to commonly used THF because in THF aldehyde and hydrate are present in approximately equal amounts, while aromatic hydrocarbons favour the aldehyde.

In general **AVL** can be converted to **desTBSDMAL** ((4R,6S)-6-(dimethoxymethyl)-4-hydroxytetrahydro-2*H*-pyran-2-one) or **AVL'** to analogous **desTBSDMAL'** ((4*R*,6S-6-(dial koxymethyl)-4-hydroxytetrahydro-2*H*-pyran-2-one) or ((4R,6S)-6-(alkylenedioxymethyl)-4-hydroxytetrahydro-2*H*-pyran-2-one) by oxidation with suitable oxidizing agent which can be selected from bromine, *N*-iodosuccinimide/tetra-*n-*butylamonium iodide in dichloromethane or NaOCl in appropriate solvent in particular with bromine in presence of week bases, in particular BaCO₃ which doesn't hydrolize the formed lactone in suitable solvent which can be polar and protic such as water at temperatures from 0 to 40 °C. The reaction proceeds well if there is an excess of bromine compared to the substrate and excess of BaCO₃ compared to the bromine. The product is conveniently isolated by extraction from water with water immiscible solvent such as ethyl acetate followed by flash chromatography.

To the **desTBSDMAL** or **des TBSDMAL'** a suitable protecting group, which can be any conventionally used protecting group, in particular silyl protecting group is introduced, in particular by reaction with *tert*-butyldimethylsilyl chloride. The reaction is conveniently done in presence of a base, selected from amines, imidazoles and pyridines, preferably imidazole in solvents selected from amides: *N,N*-dimethylformamide (DMF), *N,N-*dimetylacetamide (DMA), hexamethylphosphortriamide (HMPTA); *N*-methylpyrrolidone (NMP); *N,N*'-dimethylpropyleneurea (DMPU); *N,N,N,N*-tetramethylurea (TMU); dimethylsulfoxide (DMSO); nitriles (acetonitrile), chlorinated hydrocarbons (dichloromethane, chloroform), aromatic hydrocarbons (toluene), preferably in DMF where starting material and reagents are well soluble. The reaction can be performed at temperatures between - 10 °C to 30 °C. Preferably at 0 °C. The reaction is accomplished in period from one hour up to a day, preferably in 12 to 24 hours. The product **(DMAL** or **DMAL')** can be isolated by evaporation of chlorinated hydrocarbon solvent followed by by flash chromatography or by dilution with water and extraction to water immiscible solvent such as ethyl acetate followed evaporation when amide solvent is used for the reaction.

**DMAL** can be converted to **FL** (or **DMAL'** to **FL')** by hydrolytic cleavage of acetals by acid catalysts which do not catalyse the cleavage of lactone using Broensted or Lewis acids in organic solvent preferably Lewis acids such as FeCl₃·6H₂O, FeCl₃·SiO₂, CuCl₂·2H₂O, Zn(NO₃)₂·6H₂O, (NH₄)₂Ce(NO₃)₆, CeCl₃·7H₂O, TiCl₄, ZnBr₂, SnCl₂·2H₂O, LiBF₄ in wet acetonitrile, most preferably ZnBr₂ in methylene chloride; by transacetalization reaction with ketones in the presence of catalysts preferably with acetone in the presence of iodine; by pyridinium *p*-toluenesulfonate in wet acetone; DDQ; Montmorillonite K10; CBr₄/MeCN/H₂O; Me₃Sil.

The hydrated form of **FL** or **FL'** may used for the Wittig reaction without further purification by dissolution in appropriate solvent (toluene or dichloromethane) where dehydration to **FL** or **FL'** occurs. In the specific embodiment related to rosuvastatin in the subsequent reaction step (2*S*,4*R*)-4-(*tert-*butyldimethylsilyloxy)-6-oxo-tetrahydro-2H-pyran-2-carbaldehyde **(FL),** or **FL'** if another protecting group is used, can be reacted under condition of Wittig coupling (in the presence of base) with a ((4-(4-fluorophenyl)-6-isopropyl-2-(*N*-methylmethylsulfonamido)pyrimidin-5-yl)methyl)triphenyl-phosphonium halide or any other ((4-(4-fluorophenyl)-6-isopropyl-2-(*N*-methylmethylsulfonamido)pyrimidin-5-yl)methyl)phosphonium salt or alternatively di-i-propyl ({4-(4-fluorophenyl)-6-isopropyl-2-[methyl(methylsulfonyl)amino]-5-pyrimidinyl}methylphosphonate or any other ({4-(4-fluorophenyl)-6-isopropyl-2-[methyl(methylsulfonyl)amino]-5-pyrimidinyl}methylphosphonate ester to give *N*-(5-((*E*)-2-((2*S*,4*R*)-4-(*tert-*butyldimethylsilyloxy)-6-oxo-tetrahydro-2*H*-pyran-2-yl)vinyl)-4-(4-fluorophenyl)-6-isopropylpyrimidin-2-yl)-N-methylmethanesulfonamide **(RSVLTBS),** or an analogue if another protecting group is used. As a base lithium hexamethyldisilazane (LiHMDS), potassium hexamethyldisilazane (KHMDS), sodium hexamethyldisilazane (NaHMDS), lithium diisopropylamide (LDA), sodium hydride, butyllithium or Grignard reagents, preferably sodium hexamethyldisilazane may be used. When the source of FL is its hydrate form or a mixture FL and hydrate form thereof which is dissolved in ethers selected from THF, Et₂O, *i*-Pr₂O, *t*-BuMeO; hydrocarbons selected from: pentane, hexane, cyclohexane, methylcyclohexane, heptane; aromatic hydrocarbons selected from toluene or its chlorinated derivatives; chlorinated hydrocarbons selected from: chloroform and dichloromethane or in mixtures of these solvents, the water released from hydrate should be removed prior to the addition to the formed ylide solution. The preferred solvents for the reaction are anhydrous toluene and dichloromethane. The reaction can be performed at temperatures between - 80 °C to 40 °C. Preferably at 0 to 30 °C. The reaction is accomplished in 1 - 12 hours. Isolation of the crude product with extraction can be preformed with AcOEt, ethers or alkanes as above. Preferably with *t*-BuMeO.

The protecting group may be removed and lactone opened to produce a rosuvastatin free acid or its salt, optionally an amine, which may be converted to hemicalcium salt. The deprotection can be performed at temperatures between 0 °C to 80 °C. Preferably at 20 or 40 °C in suitable solvent, preferably a solvent selected from alcohols, acetic acid, THF, acetonitrile, methyltetrahydrofuran, dioxane, CH₂Cl₂, more preferably in alcohols and mixture of THF/AcOH. The usual deprotecting reagents may be used such as tetra-n-butylammonium fluoride, ammonium fluoride, AcCl, FeCl₃, TMSCl/HF·2H₂O, chloroethylchloroformate (CEC), Ph₃PCH₂COMeBr. Opening of lactone takes places in preferably 4:1 to 2:1 mixture of THF/H₂O as well as a pure THF at temperatures between 20 °C to 60 °C with suitable alkali such as NaOH, KOH, ammonia or amines. The hydrolysis is accomplished in 30 minutes (at 60 °C) to 2 hours (at 20 °C). After that evaporation of solvents under the reduced pressure can be conducted at temperatures between 10 °C to 50 °C and conversion to calcium salt, preferably by addition of Ca(OAc)₂×H₂O can be performed at temperatures between 0 °C to 40 °C which can be added in one portion or dropwise in 5 to 60 minutes. After the addition of Ca(OAc)₂×H₂O suspension can be stirred at temperatures between 0 °C to 40 °C from 30 minutes to 2 hours. To produce other statins the (2S,4R)-4-(tert-butyldimethylsilyloxy)-6-oxo-tetrahydro-2*H*-pyran-2-carbaldehyde (**FL**) (or in general **FL**') should be reacted under analogous condition of Wittig coupling with an appropriate reagent followed by hydrogenation when needed.

Compounds of formula 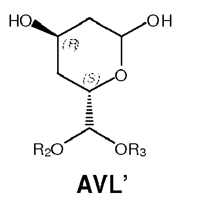 where R₂ and R₃ are independently selected from C1 - C4 alkyl, or together form a cyclic structure of formula (CH₂)ₙ where n is from 2 to 6, can be prepared as outlined on Scheme 4a from (4*R*,6*S*)-4-(*protected*)-6-(iodomethyl)-tetrahydropyran-2-one, such as (4*R*,6*S*)-4-*(tert*-butyldimethylsilyloxy)-6-(iodomethyl)-tetrahydropyran-2-one (J. Chem. Soc., Perkin Trans. 1, 1991, 133-140.) by acetate substitution, subsequent reaction with [*t-*Bu₂SnOH(Cl)]₂ and subsequent oxidation with Dess-Martin periodinane (DMP) followed by reaction with trialkyl ortoformate and reduction with di-s-butylalumminum hydride yielding (4R,6S)-4-(protected)-6-(dialkoxymethyl)tetrahydro-2*H* pyran-2-ol, from which protecting group is removed with tetra-n-butylammonium fluoride.

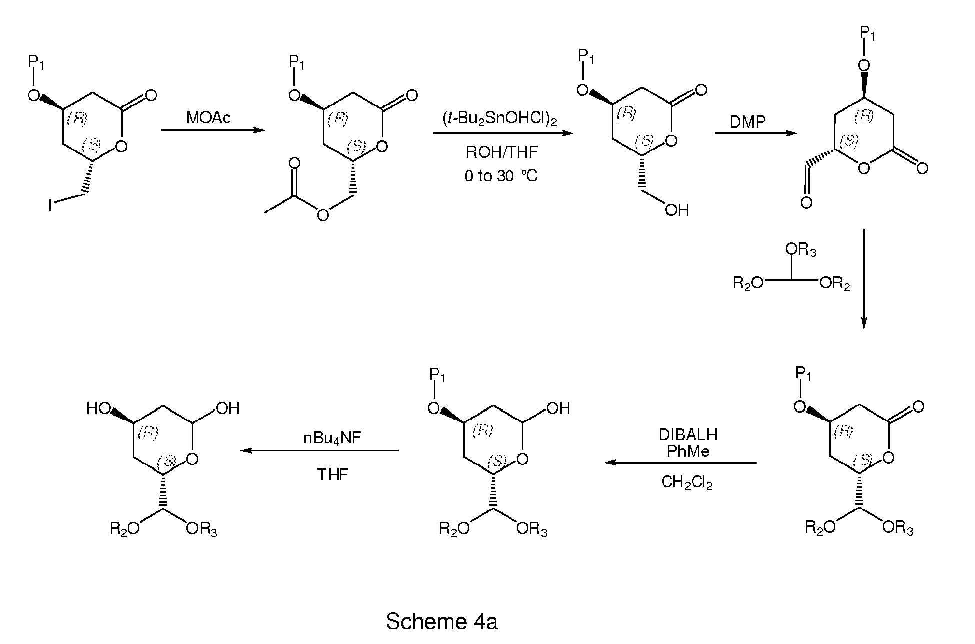

In another embodiment compound **AVL** may be prepared as outlined on Scheme 4b starting from dehydroacetic acid **P1** which is hydrolysed with 90 % sulphuric acid at 130 ºC, further oxidizing the obtained pyrone **P2** by selen dioxide in diglyme to give formyl substituted pyrone **P3.** Aldehyde is further transformed to the corresponding dimethyl acetal **P4** by reaction with methanol in the presence of gaseous hydrogene chloride, then the pyrone ring is reduced by hydrogenation in two step process first using first 10 % palladium on carbon to obtain dihydro derivative **P5** and then Raney nickel to obtain mixture of fully saturated diastereoisomers of structure **P6.** The mixture is resolved by transesterification with vinyl acetate in tetrahydrofuran in the presence of a lipase at 40 °C in which the wished 4(*R*)-6(*S*) isomer is not acetylated and finally, after removing lipase by filtration solvent by evaporation it isolated by column chromatography on silica gel using ethyl acetate - dichloromethane mixtures from other acetylated diastereoisomers. The obtained 6(*S*)-dimethoxy-4(*R*)-hydroxytetrahydropyrane-2-one is reduced by di-s-butylalumminium hydride to obtain **AVL.**

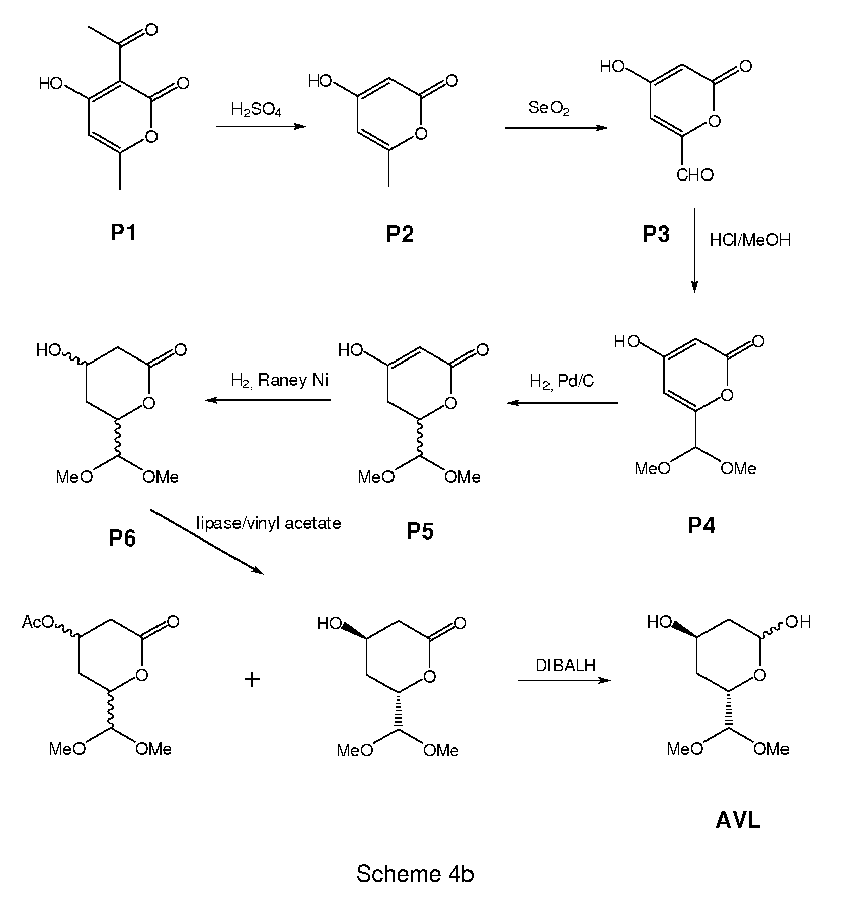

The following examples illustrate the process of the present invention and are not intended to limit the scope of the invention.

### Examples

### Example 1

### a) ((2S,4R)-4-(tert-butyldimethylsilyloxy)-6-oxo-tetrahydro-2H-pyran-2-yl)methyl acetate

To the solution of (4*R*,6*S*)-4-(*tert*-butyldimethylsilyloxy)-6-(iodomethyl)-tetrahydropyran-2-one (40.00 g, 108.0 mmol) in AcOH (660 mL) is added AgOAc (20.03 g, 118.8 mmol). The resultant mixture is then heated at 125°C for 6 hours. The reaction mixture is filtered through diatomite filter medium (Celite®). The obtained filtrate is evaporated to afford the residue. To this residue EtOAc (500 mL) and water (600 mL) are added. The organic layer is separated and the aqueous layer is washed again with EtOAc (5 × 150 mL). The combined organic layers are washed with water (4 × 300 mL), brine (5 × 300 mL) and dried over anhydrous MgSO₄, filtered and concentrated under the reduced pressure to afford 30.28 g (92.6 %) of ((2*S*,4*R*)-4-(*tert*-butyldimethylsilyloxy)-6-oxo-tetrahydro-2*H-*pyran-2-yl)methyl acetate as yellow oil (HPLC purity 98 %).
¹H NMR (300 MHz, CDCl₃) δ: 4.93 (m, 1H), 4.37 (m, 1H), 4.30 (dd, *J* = 12 Hz, *J* = 3 Hz, 1H), 4.21 (dd, *J* = 12 Hz, *J* = 5 Hz, 1H), 2.62 (d, *J* = 4 Hz , 2H), 2.11 (s, 3H), 1.84-1.80 (m, 2H), 0.89 (s, 9H), 0.09, 0.09 (2s, 6H).
¹³C NMR (75 MHz, CDCl₃) δ: 170.4, 169.1, 73.3, 65.5, 63.0, 38.9, 32.2, 20.5, 17.7, -5.1, - 5.2.

### b) (4R,6S)-4-(tert-butyldimethylsilyloxy)-6-(hydroxymethyl)-tetrahydropyran-2-one

((2*S*,4*R*)-4-(*tert*-Butyldimethylsilyloxy)-6-oxo-tetrahydro-2*H*-pyran-2-yl)methyl acetate (8.36 g, 27.64 mmol) and [*t*-Bu₂SnOH(Cl)]₂ (1.577 g, 2.764 mmol) are dissolved in MeOH/THF mixture (280 mL). The reaction mixture is stirred at 23-25°C for 27 h. After the solvent is removed under the reduced pressure the remained residue is purified by silica gel chromatography (elution with *t*-BuMeO/hexane mixture) to afford a crude product as white solid (5.59 g, 78 %). Recrystallization from n-hexane affords (3.90 g, 54 %) of (4*R*,6*S*)-4-(*tert*-butyldimethylsilyloxy)-6-(hydroxymethyl)-tetrahydro-pyran-2-one as white needles. M.p. = 102 °C (DSC peak).
¹H NMR (300 MHz, CDCl₃) δ: 4.80 (m, 1H), 4.38 (m, 1H), 3.91 (dd, *J* = 12 Hz, *J* = 3 Hz,1H), 3.66 (dd, *J =* 12 Hz, *J* = 5 Hz,1H), 2.60 (d, *J* = 4 Hz , 2H), 2.31 (bs, 1H), 1.97-1.75 (m, 2H), 0.88 (s, 9H), 0.09, 0.08 (2s, 6H).
¹³C NMR (75 MHz, CDCl₃) δ:170.1, 76.8, 64.7, 63.4, 39.2, 31.9, 25.6, 17.9, -4.9, -5.0.

### c) (4R,6S)-4-(tert-butyldimethylsilyloxy)-6-(dihydroxymethyl)tetrahydro-2H-pyran-2-one

A mixture of (4*R*,6*S*)-4-(*tert-*butyldimethylsilyloxy)-6-(hydroxymethyl)-tetrahydropyran-2-one (150 mg, 0.58 mmol) and Dess-Martin periodinane (380 mg, 0.86 mmol) in CH₂Cl₂ (15 mL) is stirred at ambient temperature for 3 hours. The mixture is diluted with *t*-BuMeO (20 mL), washed with saturated Na₂S₂O₃ solution, saturated NaHCO₃ solution, dried (MgSO₄) and concentrated to give 130 mg (87 %) of (4R,6S)-4-(tert-butyldimethylsilyloxy)-6-(dihydroxymethyl)tetrahydro-2H-pyran-2-one as white powder which is used in the next step without further purification.
¹H NMR (300 MHz, THF-d₈) δ: 5.27 (d, *J* = 6 Hz, 1H, OH), 5.19 (d, *J* = 6 Hz, 1H, OH), 4.90-4.85 (m, 1H), 4.44-4.38 (m, 2H), 2.58 (dd, *J* = 17 Hz, *J* = 4 Hz,1H), 2.44-2.36 (m, 1H), 1.92-1.87 (m, 2H), 0.91 (s, 9H), 0.10, (s, 6H).
¹³C NMR (75 MHz, THF-d₈) δ: 168.7, 91.7, 79.0, 65.1, 40.3, 31.0, 26.2, 18.7, -4.8, -4.8.

### d) (4R,6S)-4-(tert butyldimethylsilyloxy)-6-(dimethoxymethyl)tetrahydro-2H-pyran-2-one

To a solution of 1.0 g of (4*R*,6*S*)-4-(*tert*-butyldimethylsilyloxy)-6-(dihydroxymethyl)-tetrahydropyran-2-one in 100 mL of dichloromethane, a 50 mg of toluenesulfonic acid and 4.5 mL of trimethyl orthoformate are added. After 2 hours of stirring at 25°C, 0.2 g of NaHCO₃ is added and dichloromethane is distilled off. The residue is flash chromatographed (MTBE/hexane 1/1), solvents are distilled off to get the title compound.
Yield: 0.70 g of yellow, crystalline powder.
¹H NMR (300 MHz, CDCl₃) δ: 0.08 (s), 0.88 (s), 1.8 - 1.9 (m), 2.5 - 2.7 (m), 3.44 (s), 3.45 (s), 4.36 (t), 4.42 (d), 4.73 (m).
¹³C NMR (75 MHz, CDCl₃) δ: -5.0, 17.9, 25.6, 26.9, 29.7, 39.5, 55.8, 56.8, 63.3, 75.7, 105.1, 169.5.

### e) (4R,6S)-4-(tert-butyldimethylsilyloxy)-6-(dimethoxymethyl)-tetrahydro-2H-pyran-2-ol

A solution of 0.6 g of (4*R*,6*S*)-4-(*tert*-butyldimethylsilyloxy)-6-(dimethoxymethyl)-tetrahydropyran-2-one in 36 mL of dichloromethane, is cooled to - 78°C, after 2 mL of DIBALH (25 % in toluene) is added over 10 minutes. After 1 hour of stirring at -78 °C, 0.9 g of Rochelle salt in 60 mL of water is added, phases are separated and dichloromethane is distilled off. The residue is flash chromatographed (MTBE/hexane 1/1), solvents are distilled off to get the title compound.
Yield: 0.47 g of yellow oil.
Mixture of α and β anomers:
¹H NMR (300 MHz, CDCl₃) δ: 0.10 (s), 0.86 (s), 0.89 (s), 1.4-1.9 (m), 3.35 (s), 3.42 (s), 4.02 (m), 4.2 - 4.4 (m), 5.13 (d), 5.23 (d), 5.51 (d).
¹³C NMR (75 MHz, CDCl₃) δ: -5.6, -5.2, 17.7, 17.8, 25.5, 32.1, 33.2, 36.0, 40.1, 53.8, 54.2, 54.6, 55.0, 92.7, 92.8, 105.5, 105.6.

### f) (4R,6S)-6-(dimethoxymethyl)-tetrahydro-2H-pyran-2,4-diol (AVL)

To a solution of 0.47 g of (4*R*,6*S*)-4-(*tert*-butyldimethylsilyloxy)-6-(dimethoxymethyl)-tetrahydro-2*H*-pyran-2-ol in 14 mL of tetrahydrofurane, is added tetra-*n*-butylammonium fluoride in 14 mL of THF. After 15 minutes of stirring at 25 °C, the THF is distilled off. The residue is flash chromatographed (methanol). After methanol is distilled off the title compound is obtained as an oil.
Yield: 0.18 g of yellow oil.
Mixture of α and β anomers:
¹H NMR (300 MHz, CDCl₃) δ:1.4 - 1.9 (m), 3.42 (s), 3.44 (s), 3.45 (s), 4.05 (m), 4.15 - 4.35 (m), 5.20 (m), 5.25 (d).
¹³C NMR (75 MHz, CDCl₃) δ: 32.8, 35.0, 54.5, 54.5, 54.9, 55.0, 62.8, 64.5, 64.9, 92.5, 92.9, 105.4, 105.7.

### Example 2

### a) (4R,6S)-6-(dimethoxymethyl)-4-hydroxy-tetrahydropyran-2-one (desTBSDMAL)

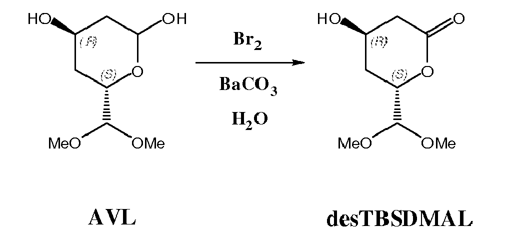

To a solution of 1.4 g of (4*R*,6*S*)-6-(dimethoxymethyl)-tetrahydro-2*H*-pyran-2,4-diol in 100 mL of water is added 2.4 g barium carbonate and 0.5 mL of bromine at 0°C. After 3 hours of stirring at 0 - 5°C, the product is extracted three times with 300 mL of ethyl acetate. Finally the ethyl acetate is distilled off. The residue is flash chromatographed (DIPE/acetonitrile 2/1), solvents are distilled off to get the title compound.
Yield: 0.7 g of yellow oil.
¹H NMR (300 MHz, CDCl₃) δ: 1.8 - 1.9 (m), 2.5 - 2.7 (m), 3.40 (s), 3.41 (s), 4.3 - 4.4 (m), 4.6-4.7 (m).
¹³C NMR (75 MHz, CDCl₃) δ: 29.3, 38.7, 55.8, 56.9, 62.4, 75.5, 105.0, 169,8.

### b) (4R,6S)-4-(tert-butyldimethylsilyloxy)-6-(dimethoxymethyl)-tetrahydropyran-2-one (DMAL)

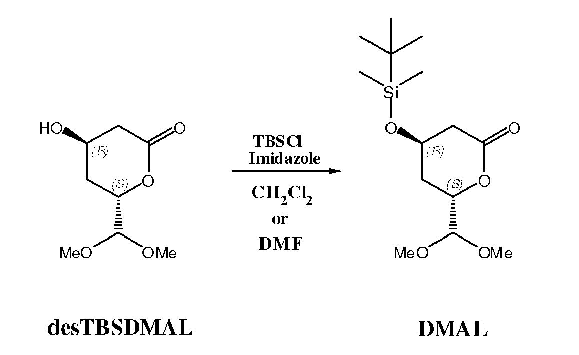

To a solution of 0.5 g of (4*R*,6*S*)-6-(dimethoxymethyl)-4-hydroxy-tetrahydropyran-2-one in 50 mL of dichloromethane, a 280 mg of imidazole is added at 25°C. After cooling to 0 °C 400 mg of *tert*-butyldimethylsilyl chloride is added. The reaction is then stirred for 18 hours and dichloromethane is distilled off. The residue is flash chromatographed (DIPE/acetonitrile 5/1), solvents are distilled off to get the title compound.
Yield: 0.40 g of yellow, crystalline powder.
¹H NMR (300 MHz, CDCl₃) δ: 0,08 (s), 0.88 (s), 1.9 (m), 2.6 (m), 3.44 (s), 3.45 (s), 4.36 (t), 4.42 (d), 4.73 (m).
¹³C NMR (75 MHz, CDCl₃) δ: -5.0, 17.9, 25.6, 26.9, 29.7, 39.5, 55.8, 56.8, 63.3, 75.7, 105.1, 169.5.

### c) (4R,6S)-4-(tert-butyldimethylsilyloxy)-6-(dihydroxymethyl)-tetrahydropyran-2-one and (2S,4R)-4-(tert-butyldimethylsilyloxy)-6-oxo-tetrahydro-2H-pyran-2-carbaldehyde

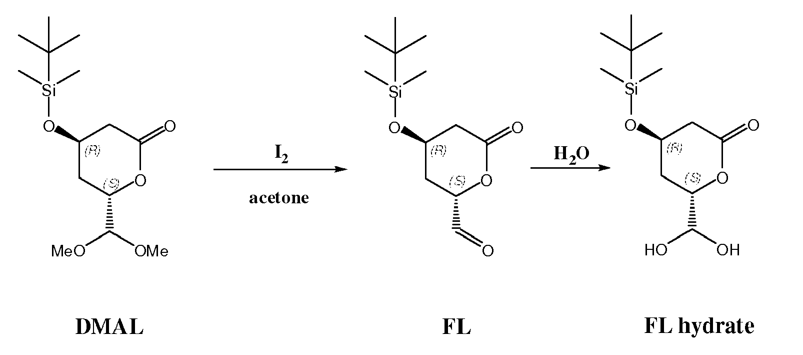

To a solution of 0.40 g of (4*R*,6*S*)-4-(*tert*-butyldimethylsilyloxy)-6-(dimethoxymethyl)-tetrahydropyran-2-one in 12 mL of acetone a 35 mg of iodine is added at 25°C. The reaction is then stirred for 48 hours and acetone is distilled off. After the 25 mL of dichloromethane is added and washed with 20 mL of saturated sodium thiosulfate solution and 20 mL of water. The organic layer is separated and the organic phase is distilled off to get the title compound.
Yield: 0.25 g of white powder.
The equilibrium between (2*S*,4*R*)*-*4*-(tert*-butyldimethylsilyloxy)-6-oxo-tetrahydro-2*H*-pyran-2-carbaldehyde and its hydrate as studied by NMR spectroscopy proves that in chlorinated hydrocarbons: chloroform, dichloromethane; hydrocarbons: pentane, hexane, heptane, methylcyclohexane and cyclohexane and particularly in aromatic hydrocarbons: toluene (or its chlorinated analogues) the equilibrium is shifted completely towards aldehyde. Use of toluene as the solvent for Wittig reaction significantly increases the yield compared to commonly used THF because in THF aldehyde and hydrate are present in approximately equal amounts, while aromatic hydrocarbons favour the aldehyde.

The isolated hydrate form of aldehyde has following NMR spectra:
¹H NMR (300 MHz, THF-d₈) δ: 5.27 (d, *J* = 6 Hz, 1H, OH), 5.19 (d, *J* = 6 Hz, 1H, OH), 4.90-4.85 (m, 1H), 4.44-4.38 (m, 2H), 2.58 (dd, *J* = 17 Hz, *J* = 4 Hz,1H), 2.44-2.36 (m, 1H), 1.92-1.87 (m, 2H), 0.91 (s, 9H), 0.10, (s, 6H).
¹³C NMR (75 MHz, THF-d₈) δ: 168.7, 91.7, 79.0, 65.1, 40.3, 31.0, 26.2, 18.7, -4.8, -4.8.

The aldehyde formed in aprotic solvent has following NMR spectra:
¹H NMR (300 MHz, CDCl₃) δ: 9.82 (s, 1H), 5.09 (dd, *J* = 11 Hz, *J* = 4 Hz,1H), 4.38 (m, 1H), 2.67 (d, *J =* 4 Hz , 2H), 2.18-2.10 (m, 1H), 1.91-1.81 (m, 1H), 0.89 (s, 9H), 0.09, (s, 6H).
¹³C NMR (75 MHz, CDCl₃) δ: 199.4, 168.0, 79.2, 62.9, 39.6, 31.4, 25.6, 17.9, -4.9.

The obtained (4*R*,6*S*)-4-(*tert*-butyldimethylsilyloxy)-6-(dihydroxymethyl)tetrahydro-2*H-*pyran-2-one is used in the next step without further purification by dissolution in toluene where dehydration to (2*S*,4*R*)-4-(*tert*-butyldimethylsilyloxy)-6-oxo-tetrahydro-2*H*-pyran-2-carbaldehyde occurs.

### Example 3

### a) N-(5-((E)-2-((2S,4R)-4-(tert -butyldimethylsilyloxy)-6-oxotetrahydro-2H-pyran-2-yl)vinyl)-4-(4-fluorophenyl)-6-isopropylpyrimidin-2-yl)-N-methylmethanesulfonamide (RSVLTBS)

To a stirred suspension of ((4-(4-fluorophenyl)-6-isopropyl-2-(*N*-methylmethyl-sulfonamido)pyrimidin-5-yl)methyl)tributylphosphonium 2,2,2-trifluoro-acetate (504 mg, 0.77 mmol) at room temperature in dry toluene (8 mL) is added sodium hexamethyldisilazane in toluene (1.3 mL of 0.6 M, 0.77 mmol) portionwise in 10 minutes. The reaction mixture is stirred for 60 min and treated at room temperature with a solution of (2*S*,4*R*)-4-(*tert*-butyldimethylsilyloxy)-6-oxo-tetrahydro-2*H*-pyran-2-carbaldehyde (200 mg, 0.77 mmol) in 25 mL of dry toluene obtained by dissolution of (4*R*,6*S*)-4-(*tert-*butyldimethylsilyloxy)-6-(dihydroxymethyl)-tetrahydropyran-2-one (214 mg, 0.77 mmol) in toluene and removal of released water. After 24 hours of stirring at room temperature the solution is treated with saturated ammonium chloride solution or water. The aqueous phase is extracted with *t*-BuMeO (2 × 20 mL), and the combined organic layers dried and concentrated. The residue is purified by silica gel chromatography (elution with t-BuMeO/hexane mixture) to give 273 mg (61 %) of *N*-(5*-*((*E*)-2-((2*S*,4*R*)-4-(*tert-*butyldimethylsilyloxy)-6-oxo-tetrahydro-2*H*-pyran-2-yl)vinyl)-4-(4-fluorophenyl)-6-isopropylpyrimidin-2-yl)-*N*-methylmethanesulfonamide as white solid.
¹H NMR (300 MHz, CDCl₃) δ: 7.62 (dd, *J*= 9 Hz, *J*= 5 Hz, 2H), 7.09 (t, *J* = 9 Hz, 2H), 6.69 (dd, *J* = 16 Hz, *J* = 1 Hz, 1H), 5.49 (dd, *J* = 16 Hz, *J* = 6 Hz, 1H), 5.22-5.16 (m, 1H), 4.29-4.27 (m, 1H), 3.56 (s, 3H), 3.50 (s, 3H), 3.32 (septet, 1H), 2.61-2.59 (m, 2H), 1.80-1.73 (m, 1H), 1.64-1.54 (m, 1H), 1.26 (d, *J* = 7 Hz , 6H), 0.87 (s, 9H), 0.07, 0.06 (2s, 6H).
¹³C NMR (75 MHz, CDCl₃) δ: 174.9, 169.5, 163.5, 163.2 (d, *J_{C-F}* = 250 Hz), 157.4, 134.7, 134.1 (d, *J_{C-F}* = 3 Hz), 132.0 (d, *J_{C-F}* = 8 Hz), 125.3, 120.5, 115.0 (d, *J_{C-F}* = 22 Hz), 75.3, 63.2, 42.3, 39.2, 36.2, 33.0, 32.1, 25.5, 21.5, 17.8, -5.0, -5.0.

### b) calcium salt of (3R,5S,E)-7-(4-(4-fluorophenyl)-6-isopropyl-2-(N-methylmethylsulfonamido)pyrimidin-5-yl)-3,5-dihydroxyhept-6-enoic acid

To a stirred solution of *N*-(5-((*E*)-2-((2*S*,4*R*)-4-(*tert*-butyldimethylsilyloxy)-6-oxo-tetrahydro-2*H*-pyran-2-yl)vinyl)-4-(4-fluorophenyl)-6-isopropylpyrimidin-2-yl)-*N-*methylmethanesulfonamide (190 mg, 0.33 mmol) in 3 mL tetrahydrofurane is added a solution of acetic acid (55 mg, 0.92 mmol) and tetra-n-butylammonium fluoride trihydrate (183 mg, 0.58 mmol) in 3 mL of tetrahydrofurane. The reaction mixture is stirred at 20 - 30 °C for 48 h, treated with 10 mL of water and extracted several times with *t-*BuMeO. The combined organic layers are washed successively with saturated NaHC0₃ solution, water and brine, dried with Na₂SO₄ and concentrated under reduced pressure. The residue is dissolved in 3 mL of a 4:1 mixture of THF/H₂O. The clear solution is warmed to 30 °C and 8.0 M NaOH (0.044 mL, 0.35 mmol) is added portionwise. The reaction mixture is stirred at 30 °C for 2 hours giving a clear yellow solution. Then THF is removed completely under the reduced pressure (20 mbar) at 40 °C. The remained water solution is diluted with H₂O to 1.5 mL and washed with AcOEt (2×1 mL). After separation from the organic layer the aqueous phase is distilled under the reduced pressure (20 mbar) at 40 °C to completely remove the dissolved AcOEt. The remained clear solution of sodium rosuvastatinate (1.3 mL) is diluted with H₂O to 1.5 mL and warmed to 40 °C. To a vigorously stirring solution of sodium rosuvastatinate is added dropwise Ca(OAc)₂×H₂O (44 mg, 0.25 mmol in 0.3 mL of H₂O) over 5 minutes at 40 °C to precipitate rosuvastatin calcium. After the complete addition the suspension is stirred further for 30 minutes at 40 °C. The white precipitate is filtered off. Then a wet white solid is suspended in H₂O (1 mL) and vigorously stirred for 1 hour at 20 °C. The undissolved precipitate is collected by filtration, washed with H₂O (1 mL) and dried in vacuum at 40 °C to give 143 mg (87 %) of rosuvastatin calcium salt as white powder.

## Claims

1. A compound of formula 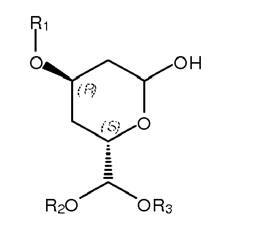 where R₁ is H or protecting group; and R₂ and R₃ are independently selected from C1 - C4 alkyl, or together form a cyclic structure of formula (CH₂)ₙ where n is from 2 to 6.

2. The compound of previous claim where R₁ is H or tert-butyldimethylsilyl, and R₂ and R₃ are methyl.

3. Process for preparing compound of formula 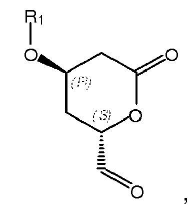 where R₁ is a protecting group comprising the following steps:
a) converting compound of formula 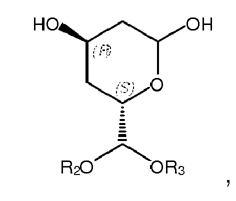 where R₂ and R₃ are independently selected from C1-C4 alkyl or together form a cyclic structure of formula (CH₂)ₙ where n is from 2 to 6,
into compound of formula: 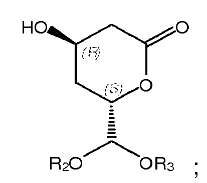 R₂ and R₃ are as described above;
b) subsequently converting said compound obtained in step a) into compound of formula: 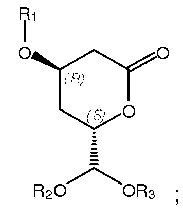 where R₁ is a protecting group, R₂ and R₃ are as described,
and;
c) subsequently cleaving acetal in compound obtained in step b).

4. The process in accordance with previous claim where R₂ and R₃ are methyl and R₁ is silyl protecting group.

5. Process for preparing the compound of formula 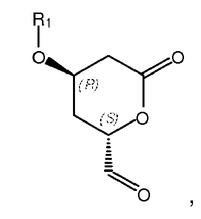 where R₁ is a protecting group,
**characterized by** providing the compound of formula 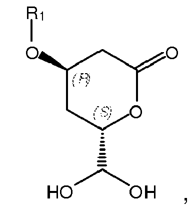 where R₁ is a protecting group,
dissolved in a solvent selected from the group of aromatic hydrocarbons, aliphatic hydrocarbons, chlorinated aromatic or aliphatic hydrocarbons and aliphatic ethers.

6. The process according to previous claim wherein the solvent is selected from toluene, heptane, methylcyclohexane, cyclohexane, hexane, pentane, dichloromethane, chloroform, 2-chlorotoluene, 3-chlorotoluene. 4-chlorotoluene, ^{t}BuMeO, Et₂O, Me₂O and THF.

7. Process for preparing a HMG CoA reductase inhibitor, **characterized in that** the compound of formula: 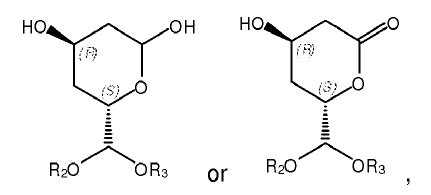 where R₂ and R₃ are independently selected from C1 - C4 alkyl or together form a cyclic structure of formula (CH₂)ₙ where n is from 2 to 6, is used.

8. Process in accordance with previous claim where HMG CoA reductase inhibitor is rosuvastatin and R₂ and R₃ are methyl.

9. Process for manufacturing of rosuvastatin **characterized in that** it comprises following steps:
a) providing compound of formula: 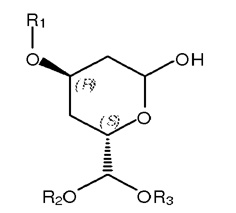 where R₁ is H or protecting group; and
R₂ and R₃ are independently selected from C1 - C4 alkyl, or together form a cyclic structure of formula (CH₂)ₙ where n is from 2 to 6;
b) converting said compound into compound of formula 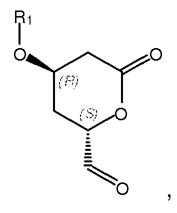 where R₁ is a protecting group; and
c) reacting said compound obtained in step b) under condition of Wittig coupling with compound of formula 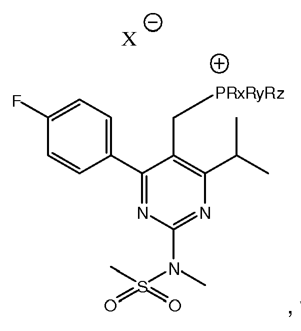
wherein Rx, Ry, and Rz, are the same or different and are selected from optionally substituted C₁- C₈ alkyl or C₃-C₆ cycloalkyl or C₁- C₈ alkenyl or C₅-C₆ cycloalkenyl or aryl,
and X is an anion, preferably halogen or RCOO- anion, more preferably chloride, bromide or trifluoroacetate;
to give a compound of formula: 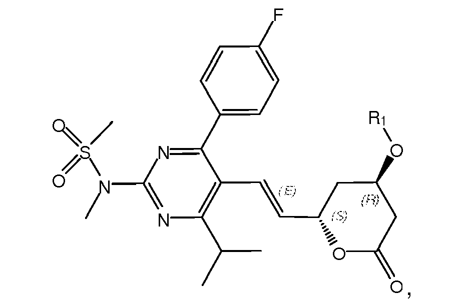 and subsequently converting said compound to rosuvastatin or its salt.

10. Use of any of the compounds of formula 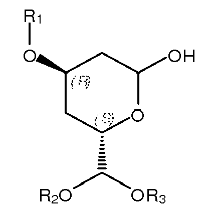 where R₁ is H or protecting group; and
R₂ and R₃ are independently selected from C1 - C4 alkyl
for the synthesis of compound of formula 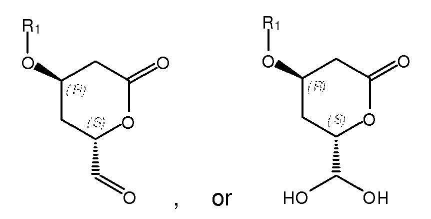 where R₁ is H or a protecting group.
